# FASCICULE DE BREVET EUROPEEN

(11) **EP 1 406 684 B1**
(45) Date de publication et mention de la délivrance du brevet: **19.09.2012**
(21) Numéro de dépôt: 02791497.7
(22) Date de dépôt: 12.07.2002
(51) Int. Cl.: A61M 1/02

(54) **PROCEDE DE FIABILISATION DE LA TRACABILITE DES PRELEVEMENTS SANGUINS**
VERFAHREN ZUR SICHERUNG DER VERFOLGUNG VON BLUTKONSERVEN
METHOD FOR MAKING ENHANCING THE RELIABILITY OF THE TRACEABILITY OF BLOOD SAMPLES

(30) Priorité: 12.07.2001 FR 0109246
(43) Date de publication de la demande: 14.04.2004
(73) Titulaire: Biolog, 92517 Boulogne Billancourt (FR)
(72) Inventeur: DE GAULLE, Antoine, F-75018 Paris (FR); MONGRENIER, Jean-Claude, F-78100 Saint Germain en Laye (FR)
(74) Mandataire: Schmitt, John
(86) Numéro de dépôt international: PCT/FR2002/002465
(87) Numéro de publication internationale: WO 2003/011364

(56) Documents cités:
- EP-A- 0 639 384
- FR-A- 2 796 182
- US-A- 6 113 554

## Description

La présente invention concerne un procédé de fiabilisation de la traçabilité des prélèvements sanguins notamment lors des opérations d'extraction du plasma et des globules rouges.

Le prélèvement du sang total sur un donneur se fait dans une poche de sang mère qui est reliée au donneur par un cathéter enfoncé dans une veine, lui-même relié par une première tubulure souple à ladite poche de sang mère posée sur un peson-agitateur qui permet de prélever une quantité de sang total prédéterminée. Cette poche de sang mère est reliée à d'autres poches dans des configurations variables en fonction des caractéristiques du donneur en vue d'extraire notamment le plasma et les globules rouges et éventuellement les plaquettes ; chaque configuration de poches constitue ce qui est appelé ci-après un "kit de prélèvement".

Il y a de nombreux kits de prélèvement différents qui évoluent en fonction des habitudes de travail des besoins et/ou de la réglementation ; dans ce qui suit on décrit un kit de prélèvement servant d'appui aux explications concernant la fiabilisation de la traçabilité des poches de sang : ces explications restent transposables pour les kits de prélèvement différents de celui décrit ci-dessous.

Ladite poche de sang mère décrite ci-dessus est, par exemple, reliée par une deuxième tubulure souple, à un premier filtre à leucocytes comportant un bipasse qui permet de le contourner si le traitement ultérieur prévoit d'extraire les plaquettes ; le premier dispositif de filtration est relié, par une troisième tubulure souple, à une poche de sang primaire ; la poche de sang primaire est à son tour reliée à une quatrième tubulure souple qui se divise en une cinquième tubulure souple, intégrant un deuxième dispositif de filtration, reliée à une poche à plasma et à une sixième tubulure souple reliée à une poche d'anticoagulant ; la quatrième tubulure est raccordée du même côté de la poche de sang primaire que la troisième tubulure. Les poches à plasma et d'anticoagulant comportent des détrompeurs qui servent au moment de la séparation des phases.

Dans ce qui suit on va décrire un processus théorique de traitement du sang total prélevé servant uniquement d'appui aux explications.

Une fois le sang total prélevé dans la poche de sang mère, la première tubulure est fermée par une soudure et la poche de sang mère, séparée du cathéter, est suspendue en hauteur de manière que le premier dispositif de filtration et la poche de sang primaire soient suspendus à la poche de sang mère ; la poche à plasma et la poche d'anticoagulant peuvent indifféremment être suspendues ou non à la poche de sang primaire et la quatrième tubulure est obturée pour empêcher le sang filtré de pénétrer dans la poche à plasma. Les deuxième et troisième tubulures sont alors ouvertes et le sang contenu dans la poche de sang mère peut alors s'écouler à travers le premier dispositif de filtration et s'accumuler dans la poche de sang primaire.

Une fois le sang filtré, la troisième tubulure est fermée par une soudure et l'ensemble constitué par le premier dispositif de filtration et la poche de sang mère est séparé de la poche de sang primaire.

La poche de sang primaire, la poche à plasma et la poche d'anticoagulant sont alors placées dans une centrifugeuse ; cette opération consiste tout d'abord à placer dans chacun de deux conteneurs de centrifugation en matière plastique rigide à ouverture totale, une poche de sang primaire, avec les raccordements de la troisième et quatrième tubulure souple tournés vers le haut du conteneur et ses poches à plasma et d'anticoagulant ; ensuite chaque conteneur est placé sur l'un des deux plateaux d'une balance pour égaliser leur poids avec précision afin de les rendre centrifugeables ; chaque conteneur centrifugeable est placé dans un des deux godets de centrifugation d'une centrifugeuse : l'opération de centrifugation se fait suivant un processus prédéterminé et les conteneurs centrifugés sont extraits des godets de centrifugation et les poches de sang primaires centrifugées et les poches à plasma et d'anticoagulant sont extraites de leur conteneur de centrifugation ; les poches de sang primaires centrifugées ayant présenté des fuites pendant cette opération sont éliminées ; le contenu des autres poches de sang primaires centrifugées présentent deux phases principales qui sont, d'une part, une phase riche en globules rouges accumulée dans le fond de la poche de sang primaire et, d'autre part, une phase constituée de plasma qui surnage ; les poches de sang primaires et secondaires sont introduites dans un séparateur.

Le séparateur comporte trois berceaux de réception des poches de sang ; un premier berceau est destiné à recevoir la poche de sang primaire, un deuxième berceau reçoit la poche à plasma et un troisième berceau la poche d'anticoagulant ; le premier berceau est placé en dessous des deux autres berceaux ; les trois berceaux forment chacun une cavité fermée de forme générale parallèlépipédique comportant deux grandes faces principales qui sont, d'une part une porte plane occupant sensiblement la surface d'une grande face et ouvrant sur l'extérieur du séparateur, d'autre part une face plane mobile susceptible de se déplacer parallèlement à elle-même suivant un axe perpendiculaire au plan des grandes faces.

La poche de sang primaire est mise verticalement avec la phase riche en globules rouges en bas dans le premier berceau dont la paroi mobile, verticale, est suffisamment reculée pour la contenir en entier et la porte est refermée ; la poche à plasma qui est destinée à la réception du plasma est placée dans le deuxième berceau qui comporte notamment un gabarit empêchant d'y mettre, à sa place, la poche d'anticoagulant ; la poche d'anticoagulant contient de l'anticoagulant destiné à la phase riche en globules rouges ; cette dernière est placée dans le troisième berceau, comportant lui-même un gabarit dont la paroi mobile est reculée pour contenir la poche d'anticoagulant ; la cinquième tubulure traverse un premier dispositif de fermeture, situé entre le deuxième dispositif de filtration et la poche à plasma permettant, soit de fermer provisoirement la cinquième tubulure par pincement, soit de la fermer par soudure-coupe en séparant la poche de sang primaire de la poche à plasma contenant le plasma ; cette opération se fait en coupant de telle sorte que la tubulure soit fermée par soudure aussi bien vers la poche de sang primaire que vers la poche à plasma ; de même, la sixième tubulure traverse un deuxième dispositif de fermeture qui, comme précédemment décrit, soit pince la sixième tubulure, soit ferme cette dernière par soudure et sépare la poche d'anticoagulant de la poche de sang primaire.

À la mise en route du séparateur après le positionnement de la poche de sang primaire, de la poche à plasma et de la poche d'anticoagulant, le deuxième dispositif de fermeture pince la sixième tubulure tandis que la cinquième tubulure est ouverte sur toute sa longueur ; la paroi mobile du deuxième berceau, manoeuvrée par un vérin, vient en appui sur la poche à plasma, tandis que la paroi mobile du premier berceau est mue par un vérin pour comprimer suffisamment la poche de sang primaire pour faire remonter le plasma à travers le deuxième dispositif de filtration vers la poche à plasma qui se gonfle progressivement en refoulant la paroi mobile du deuxième berceau ; un dispositif de contrôle optique traversé par la quatrième tubulure détecte l'arrivée de la phase riche en globules rouges, ce qui déclenche une nouvelle opération consistant notamment dans la fermeture de la cinquième tubulure et sa soudure-coupe suivi de l'ouverture de la sixième tubulure permettant le transfert du contenu de la poche d'anticoagulant vers la poche de sang primaire par compression de cette dernière par la paroi mobile du troisième berceau et le retrait de la paroi mobile du premier berceau ; la sixième tubulure est à son tour soudée et coupée afin que la poche d'anticoagulant soit séparée de la poche de sang primaire. La phase riche en globules rouges reste dans la poche primaire et la sixième tubulure est soudée et coupée pour éliminer la poche d'anticoagulant qui reste vide.

Au cours de ces diverses opérations les informations doivent être complétées des informations concernant chaque opération et transférées dans leur intégralité à plusieurs reprises ; les informations concernant la poche de sang mère complétée des informations concernant la filtration doivent être entièrement transférées sur la poche de sang primaire puisque la poche de sang mère et le premier dispositif de filtration sont supprimés après la fermeture par soudure et la coupe de la troisième tubulure ; les informations spécifiques concernant la filtration à transférer sont, par exemple, l'identité des intervenants, les références de l'appareillage support, la durée de l'opération et, par exemple, la date de fin d'opération (la date est ici entendue comme comportant le jour et l'heure), les incidents éventuels. Les conditions dans lesquelles la centrifugation s'est réalisée sont inscrites sur la poche de sang primaire ; elles concernent, par exemple, le numéro de la machine, l'identité de l'opérateur, la vitesse de centrifugation et sa durée et éventuellement les incidents survenus en cours d'opération. La séparation de la phase riche en globules rouges et du plasma implique aussi l'introduction de nouvelles informations sur la poche à plasma venant de la poche de sang primaire et des informations concernant l'opération de séparation et aussi de nouvelles informations sur la poche de sang primaire concernant la poche d'anticoagulant et l'opération de séparation.

La multiplicité des retranscriptions d'informations, l'inscription sur la poche de sang concernée des informations nouvelles après chaque opération est source d'erreur susceptible de rendre inopérant la traçabilité des prélèvements sanguins.

Un dispositif de traçabilité des prélèvements sanguins, selon le brevet EP 1 072 030, est en cours de développement ; il associe une puce électronique à la poche de sang ; chaque puce électronique comporte une antenne annulaire qui communique avec l'antenne annulaire d'un dispositif de communication électronique relié à un dispositif informatique susceptible de fournir à la puce électronique, d'une part de l'énergie et d'autre part des informations qu'elle mémorise et qu'elle est susceptible de restituer audit dispositif informatique par l'intermédiaire du dispositif de communication électronique ; une puce électronique mère est fixée sur la poche de sang mère et recueille toutes les informations sur le donneur et les résultats des analyses permettant la qualification de la poche de sang mère ; la puce électronique mère est, par exemple, fixée sur un support de puce souple de forme, par exemple, rectangulaire de quelques centimètres de côté sur lequel est imprimé un circuit métallisé en boucles formant l'antenne annulaire de communication ; dans une version préférée de l'invention, le support de puce souple de la puce électronique mère est placé sur une des grandes faces de la poche de sang mère et sous une étiquette rectangulaire recouvrant la plus grande partie d'une face principale. La puce électronique primaire équipant la poche de sang primaire est placée sous une étiquette recouvrant la grande face mais à un endroit différent par rapport à la poche de sang mère de manière que, lorsque les poches de sang mère et primaire sont superposées, les puces mère et primaire ne le soient pas. Il en est de même des premières et deuxièmes puces électroniques équipant les poches à plasma et d'anticoagulant. De préférence, les supports de puce sont toujours placés au même endroit par rapport à l'étiquette de manière à faciliter le positionnement de l'antenne du dispositif de communication électronique permettant d'enregistrer dans la puce électronique mère de la poche de sang mère les caractéristiques du donneur de sang et les conditions de prélèvement.

Le document FR 2 796 182 décrit un dispositif de transmission de données entre une puce électronique associée à une poche de sang et le dispositif informatique d'un dispositif de pesage utilisé pour les prélèvements de sang et de plasma. Ce dispositif de transmission de données est constitué d'une antenne d'encodage, en forme de boucle, qui est intégrée dans un des éléments constitutifs du dispositif de pesage, ledit dispositif de pesage étant muni d'un plateau de pesage oscillant sur lequel vient se fixer un support de poche de sang dans lequel est déposée la poche de sang équipée d'une puce électronique munie d'une antenne en forme de boucle. L'antenne d'encodage est ainsi intégrée dans la paroi du support de poche de sang et elle est reliée par un cordon au dispositif informatique associé au dispositif de pesage. Les informations liées au prélèvement sont d'abord recueillies au niveau dudit dispositif informatique et transférées dans la puce électronique avant que la poche de sang ne soit enlevée du support de poche de sang éliminant ainsi le risque d'erreur d'une transmission d'informations, relatives au prélèvement, postérieurement à la prise de sang. L'antenne d'encodage, après avoir transmis les informations liées au prélèvement dans la puce électronique, interroge ensuite la puce électronique pour comparer les informations reçues par la puce électronique aux informations émises vers la puce électronique afin de contrôler qu'elles ont été correctement transmises.

L'objet de l'invention consiste dans l'adaptation des équipements utilisés lors des opérations de filtration, centrifugation, séparation, de manière à permettre la fiabilisation de la traçabilité des prélèvements sanguins effectués dans des poches de sang équipées de puces électroniques.

Sur les dessins annexés:
La figure 1 représente un schéma de principe de la fiabilisation de la traçabilité lors des diverses phases de la filtration.
La figure 2 représente un schéma de principe de la fiabilisation de la traçabilité lors des diverses phases de la centrifugation ; un conteneur de centrifugation y est représenté en vue éclatée.
La figure 3 représente un schéma de principe de la fiabilisation de la traçabilité lors des diverses phases de la centrifugation.
La figure 4A représente un crochet de suspension au poste d'accrochage des poches de sang mères à filtrer.
La figure 4B représente le crochet de suspension de la figure 4A au poste de déchargement des poches de sang mères vides après filtration.

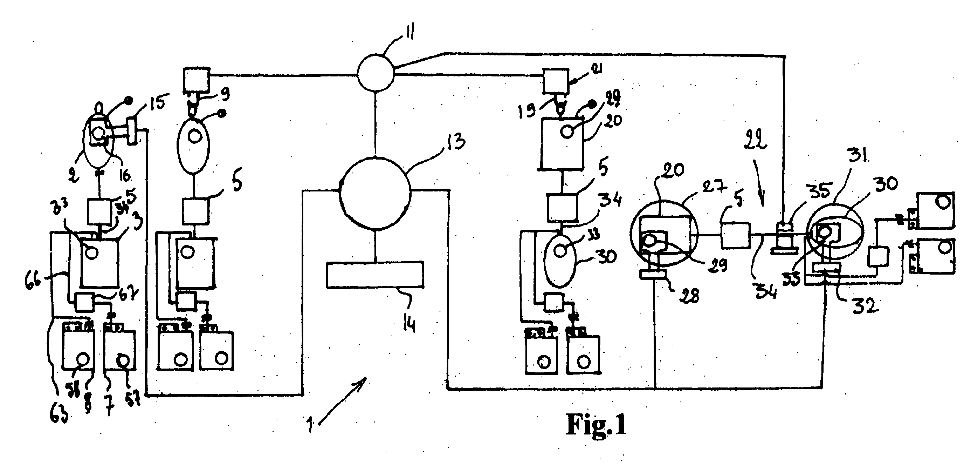

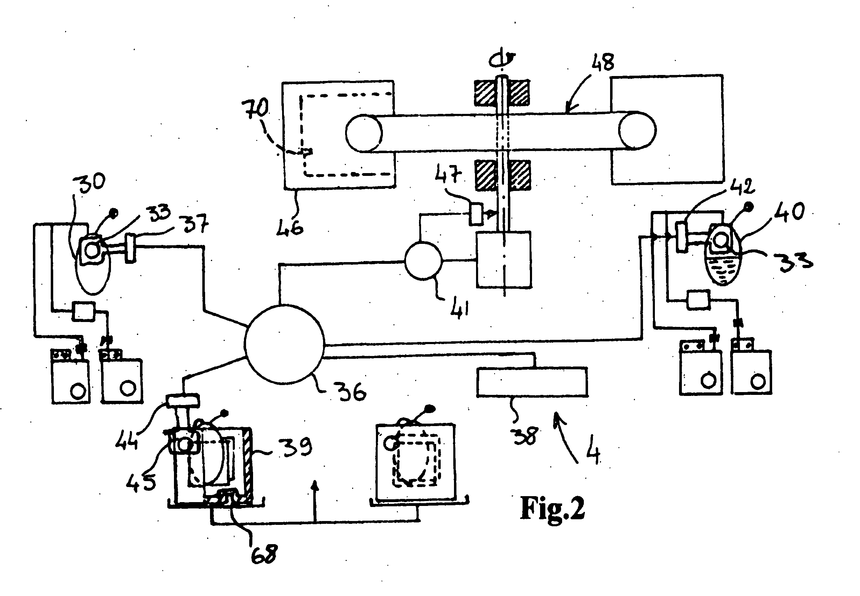

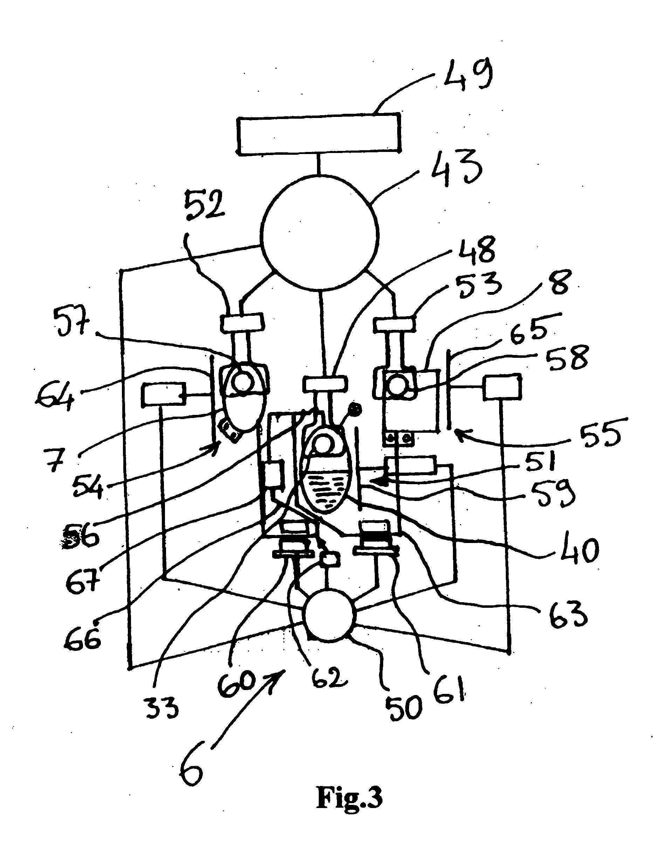

L'invention consiste à intégrer dans le processus de chaque machine de filtration, centrifugation et séparation, des moyens informatiques de recueil et de traitement d'informations, concernant la traçabilité des prélèvements sanguins, provenant de la poche de sang à traiter et provenant de la machine elle-même et de ses opérateurs ; ces moyens informatiques sont associés à des moyens de recueil d'informations et à des moyens de transfert d'informations vers la ou les poches de sang contenant le produit sanguin transformé, complétés par des moyens de fiabilisation concernant notamment la manutention ; le recueil d'informations consiste en début d'opération à recueillir les informations contenues dans la puce électronique de la poche de sang à traiter et à les compléter des informations concernant les conditions de traitement qui sont, par exemple, recueillies auprès de l'automate commandant la machine et notamment, par exemple, la date, le numéro de la machine et ses paramètres de fonctionnement, lesdites informations étant, d'autre part, introduites par l'opérateur qui, par exemple, décline son identité soit par le biais d'un badge, soit par l'intermédiaire d'un clavier. Le transfert d'informations concernant le dispositif de filtration 1 (fig.1) se fait ver la poche de sang primaire 3 lorsqu'elle est remplie de sang filtré à travers un premier dispositif de filtration 5, en provenance initiale de la poche de sang mère 2 ; le transfert d'informations concernant le dispositif de centrifugation 4 (fig.2) se fait vers la poche de sang primaire centrifugée 40 qui était avant centrifugation la poche de sang primaire 30 filtrée ; le transfert d'informations concernant le dispositif de séparation 6 (fig.3) en provenance de la poche de sang primaire centrifugée 40 et de la poche d'anticoagulant 8 se fait, d'une part ver la poche à plasma 7 lorsqu'elle est remplie de plasma et, d'autre part, vers la poche de sang primaire centrifugée 40, lorsqu'elle ne contient plus que la phase riche en globules rouges ; les moyens de recueil et de transfert d'informations sont complétés par des moyens de fiabilisation qui consistent à garantir, d'une part la transmission des informations à la bonne poche destinataire et, d'autre part, la transmission de l'ensemble des informations nécessaires pour garantir une bonne traçabilité.

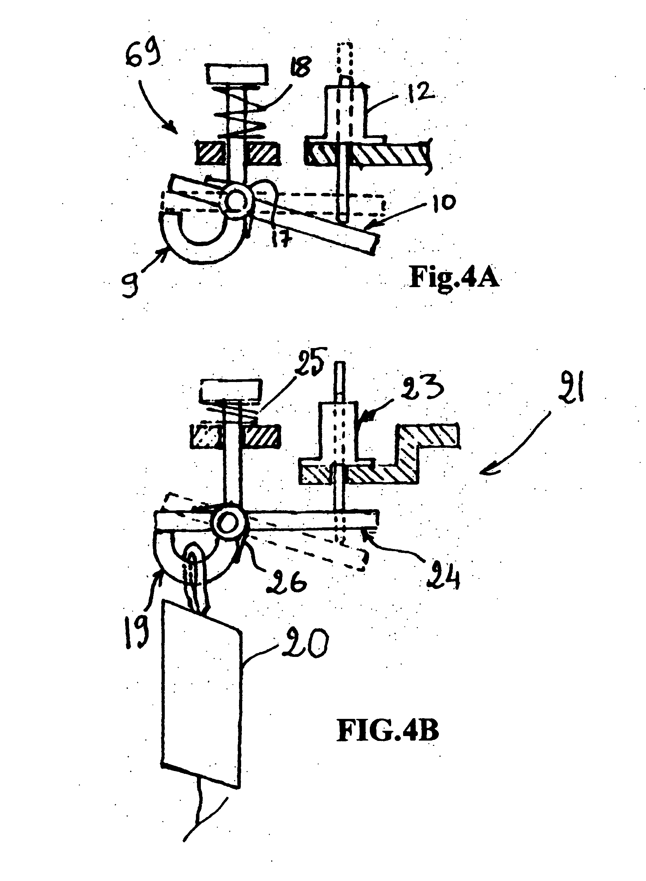

En ce qui concerne le dispositif de filtration 1 (fig.1), un moyen de procéder consiste, par exemple, à disposer de crochets de suspension 9, des poches de sang mère 2, solidaires d'une machine de filtration qui est chargée de gérer la filtration ; le nombre de crochets 9 est déterminé en fonction du nombre de poches à traiter quotidiennement ; les crochets de suspension 9 peuvent être fixes ou solidaires d'un carrousel ; par exemple, le crochet de suspension 9 (fig.4A) comporte un dispositif d'ouverture et de fermeture 10 fonctionnant, par exemple, à l'aide d'un premier dispositif électromécanique 12 commandé depuis un dispositif de pilotage électronique 11 (fig.1) solidaire de la machine de filtration. Au repos les crochets de suspension 9 sont fermés et il est impossible d'y suspendre une dose mère ; la machine dispose, par exemple, d'un premier dispositif informatique de gestion 13, spécifique du dispositif de filtration 1, accessible par un clavier 14, permettant notamment d'entrer dans la machine de filtration des paramètres permanents de fonctionnement tels que, par exemple, la date, le numéro de la machine, les conditions à remplir pour ouvrir ou fermer un crochet de suspension 9. ainsi que des paramètres variables tels que, par exemple, l'identification de l'opérateur ou des informations concernant un incident de filtration ; un dispositif de communication électronique 15, manipulé par un opérateur, est relié au premier dispositif informatique de gestion 13 ; l'opérateur lit avec le premier dispositif de communication électronique 15 le contenu de la puce mère 16 de la poche de sang mère 2 et l'enregistre dans le premier dispositif informatique de gestion 13, rajoute la date de début de l'opération de filtration et affecte un crochet de suspension 9 à la poche de sang mère 2 concernée ; le crochet 9 (fig.4A) désigné s'ouvre, par exemple, par action du premier dispositif électromécanique 12 commandé par le premier dispositif informatique de gestion 13 (fig.1) via le dispositif de pilotage électronique 11, lorsque, par exemple le crochet de suspension 9 arrive à un poste d'accrochage 69 ; l'ouverture se fait, par exemple, par basculement du dispositif d'ouverture et de fermeture 10 (fig.4A) qui tend un ressort hélicoïdal 17 ; lorsque l'opérateur accroche la poche de sang mère 2 sur le crochet de suspension 9 (fig.4A) désigné, le poids de cette dernière entraîne la refermeture du crochet de suspension 9 par abaissement de ce dernier par compression du ressort à boudin 18 et détente du ressort hélicoïdal 17 qui rabat le dispositif d'ouverture et de fermeture 10 ; un contacteur de fin de course indique alors que la poche de sang mère est bien accrochée ; lorsque la filtration est finie l'opérateur qui l'a constatée visuellement donne l'ordre d'ouvrir le crochet de suspension 19 (fig.4B) concerné soit en passant par le clavier 14 (fig.1) soit par une commande spécifique placée au voisinage du crochet de suspension 19 et une nouvelle date est inscrite dans le premier dispositif informatique de gestion 13 ; le crochet de suspension 19 (fig.1 et fig.4B) s'ouvrant au poste de déchargement 21, par exemple, sous l'action d'un deuxième dispositif électromécanique 23 appuyant sur le dispositif d'ouverture et de fermeture 24 en mettant le ressort hélicoïdal 26 sous tension ; la poche de sang mère vide 20 est enlevée lorsqu'elle arrive au poste de déchargement 21 ; elle est placée sur un appareillage de soudure-coupe 22 tandis que le crochet de suspension 19 est refermé ; il est refermé, par exemple, par retrait du deuxième dispositif électromécanique 23 et détente du ressort hélicoïdal 26 au niveau du poste de déchargement 21 et échappement et remontée du crochet de suspension 19 sous l'action du ressort à boudin 25 comprimé agissant lorsque le crochet de suspension 19 quitte le poste de déchargement 21 et remonte ; un contact de fin de course indique que la poche de sang mère a été déchargée et lorsqu'elle a été séparée de la poche de sang primaire et les informations correctement transmises à cette dernière, le crochet 19 est mis en attente d'une nouvelle affectation.

L'appareillage de soudure-coupe 22 (fig.1) comprend un premier dispositif de soudure-coupe 35 qui assure la fermeture par soudure de la troisième tubulure 34 et la séparation de la poche de sang mère 20 vide avec son premier dispositif de filtration 5 et la poche de sang primaire 30 par une coupe située au milieu de la soudure ; l'appareillage de soudure-coupe 22 comporte en outre, par exemple, un premier alvéole 27, dans lequel on place la poche de sang mère vide ; le premier alvéole 27 est muni d'un deuxième dispositif de communication électronique 28 qui lit à nouveau le contenu de la puce électronique mère 29 de la poche de sang mère 20 vide et identifie dans le premier dispositif informatique de gestion 13 la poche de sang mère 2 concernée ; la poche de sang primaire 30 est placée dans un deuxième alvéole 31 muni d'un troisième dispositif de communication électronique 32 qui enregistre dans la puce électronique primaire 33 les données contenues dans le premier dispositif informatique de gestion 13 concernant ce prélèvement ; la troisième tubulure 34 est introduite dans le dispositif de soudure-coupe 35 ; ensuite lorsque toutes les informations ont été correctement transmises le dispositif informatique de gestion 13 donne l'ordre au dispositif électronique de pilotage 11 au premier dispositif de soudure-coupe 35 d'effectuer la soudure-coupe en faisant la coupe au milieu de la soudure.

En ce qui concerne la fonction de centrifugation, le dispositif de centrifugation 4 (fig.2) est équipé d'un deuxième dispositif informatique de gestion 36 relié à un quatrième dispositif de communication électronique 37, à un clavier 38 et susceptible de recevoir en direct toutes les informations concernant les conditions de centrifugation. Avant l'introduction de chacune des deux poches de sang primaires 30, dans un des conteneurs de centrifugation 39, les puces électroniques primaires 33 sont lues par le quatrième dispositif de communication électronique 37 et les informations contenues sont stockées totalement ou partiellement dans le deuxième dispositif informatique de gestion 36 ; en effet, comme les informations issues de la centrifugation doivent être retournées dans la même poche de sang primaire 30 qui a été centrifugée et qu'on appelle ci-après "poche de sang primaire centrifugée 40", on peut ne prendre que des informations permettant d'identifier la poche de sang primaire 30 ; ensuite la poche de sang primaire 30 est introduite dans le conteneur de centrifugation 39 qui comporte une puce électronique de conteneur 45 constituant un moyen d'identification, intégrée, par exemple, dans sa paroi latérale qui contient des critères d'identification spécifiques au conteneur de centrifugation considéré et dans laquelle un cinquième dispositif de communication électronique 44 introduit les caractéristiques de la poche de sang primaire 30 qu'il contient. De préférence, la poche de sang primaire 30 comporte un détrompeur permettant d'orienter la poche de sang de manière que le raccordement de la troisième et quatrième tubulures soit situé en haut du conteneur ; le conteneur de centrifugation 39 peut aussi comporter un détrompeur 68 constituant un moyen d'identification et qui oblige l'opérateur à le placer systématiquement dans le même godet de centrifugation 46 de la centrifugeuse 48 ; le détrompeur peut être constitué d'un ergot 70 placé au fond du godet de centrifugation 46 et venant se loger dans le détrompeur 68 constitué d'une cavité pratiquée dans le fond du conteneur de centrifugation 39 ; l'information concernant l'affectation du godet de centrifugation 46 au conteneur de centrifugation 39 est inscrite dans la puce électronique de conteneur 45 de manière que les informations concernant le godet de centrifugation 46 soient orientées vers la poche de sang centrifugée 40 concernée au moment du transfert d'informations après centrifugation ; ensuite l'opérateur introduit dans le deuxième dispositif informatique de gestion 36, par exemple par le clavier 38, des informations variables telles que son identité ou la relation d'incidents tels que la double centrifugation lorsque la poche de sang primaire transfusée 40 est mise à l'envers dans son conteneur 39. Lorsque l'ensemble des informations que doit avoir le deuxième dispositif informatique de gestion 36 est disponible et enregistré, le cycle de centrifugation peut être exécuté : les paramètres affichés de l'opération de centrifugation et les caractéristiques réelles de la centrifugation sont fournis par le dispositif électronique de pilotage 41 et enregistrés directement dans le deuxième dispositif informatique de gestion 36 ; dans ce dernier, on enregistre, par exemple, la vitesse de rotation en fonction du temps, grâce à un dispositif de mesure de la vitesse 47 ce qui permet d'en déduire, après intégration mathématique, un indice de centrifugation. Après la centrifugation, un sixième dispositif de communication 42 est utilisé pour identifier la poche de sang primaire centrifugée 40 concernée et le conteneur de centrifugation 39 qui la contient afin de lui réintroduire, par exemple, les informations nouvelles concernant les conditions de centrifugation et notamment les informations spécifiques au godet de centrifugation 46 ayant contenu la poche de sang primaire centrifugé 40. Bien que dans la description les dispositifs de communication électronique 37,44,42 soient chaque fois présentés comme étant différents, un même dispositif de communication électronique peut servir à plusieurs postes en fonction de sa programmation ; cette observation est bien évidemment valable pour les postes de filtration et de séparation. Pour améliorer la fiabilité de l'opération, des sécurités sont placées qui, par exemple, bloquent le démarrage de la centrifugeuse si au moins une des informations n'a pas été recueillie ou transmise par le deuxième dispositif informatique de gestion 36.

La fonction de séparation est constituée d'un dispositif de séparation 6 (fig.3) qui comporte aussi un troisième dispositif informatique de gestion 43 relié à un septième dispositif de communication électronique 48, à un clavier 49 et. par exemple, à un dispositif de pilotage électronique 50 commandant le cycle de séparation ; l'opérateur entre, par exemple, par le clavier 49 son identité et ensuite à l'aide du septième dispositif de communication électronique 48 recopie la totalité des informations contenues dans la poche de sang primaire centrifugée 40 pour les stocker dans le troisième dispositif informatique de gestion 43 ; les informations résultant du cycle de séparation des phases sont intégrées dans le troisième dispositif informatique de gestion 43 puis transmis, d'une part vers la poche à plasma 7 contenant la phase de plasma, d'autre part vers la poche de sang primaire centrifugée 40 qui ne contient plus que la phase riche en globules rouges dont une partie provient de la poche d'anticoagulant 8.

Un moyen de transférer ces informations consiste à installer le septième dispositif de communication électronique 48 dans la porte plane du premier berceau 51 de manière à ne pouvoir recopier le contenu de la puce de la poche primaire centrifugée 40 que lorsque cette dernière est en place pour la séparation ; de même, un huitième et un neuvième dispositifs de communication électronique 52 et 53, reliés au troisième dispositif informatique de gestion 43, sont placés respectivement dans la porte du deuxième et du troisième berceaux 54 et 55.

Les quatrième, cinquième et sixième tubulures 56,66,63 sont débarrassées des dispositifs d'obturation et placées dans un deuxième et troisième dispositifs de soudure-coupe 60 et 61 qui sont aussi capables d'effectuer un simple pincement de la tubulure.

Un dispositif de détection optique 62 est placé sur la quatrième tubulure 56. La quatrième tubulure est laissée ouverte et la sixième tubulure 63 est pincée par le troisième dispositif de soudure-coupe 61.

Dans un premier temps, la paroi mobile 59 du premier berceau 51 refoule le plasma qui est envoyé dans la poche à plasma 7, en traversant le deuxième dispositif de filtration 67 de la cinquième tubulure 66, faisant reculer la paroi mobile 64 du deuxième berceau ; lorsque le dispositif de détection optique 62 détecte l'arrivée de la phase riche en globules rouges, le mouvement de la paroi mobile 59 est bloqué et la poche à plasma 7 est alors refermée et séparée de la poche de sang primaire centrifugée 40 par le deuxième dispositif de soudure-coupe 60 ; le troisième dispositif informatique de gestion 43 renseigne la première puce électronique 57 ; puis le troisième dispositif de soudure-coupe 61 libère le passage dans la sixième tubulure 63 et la paroi mobile 65 du troisième berceau 55 commence à refouler l'anticoagulant dans la poche de sang primaire centrifugé 40. en refoulant la paroi mobile 59 du premier berceau 51, jusqu'à ce qu'elle s'arrête en fin de course ; le troisième dispositif de soudure-coupe 61 ferme la sixième tubulure 63 et sépare la poche d'anticoagulant 8 de la poche de sang primaire centrifugée 40 qui contient la phase riche en globules rouges ; la deuxième puce électronique 58 de la poche d'anticoagulant 8 est alors interrogée par le neuvième dispositif de communication électronique 53 juste avant le processus de soudure-coupe et les informations sont transférées dans la puce électronique primaire 33. Le cycle étant terminé la poche à plasma 7 et la poche de sang primaire centrifugée 40 contenant la phase riche en globules rouges peuvent être enlevées de leurs berceaux 54 et 51. À chaque phase de transfert d'informations le cycle de la machine est bloqué tant que les informations attendues ou transmises par le troisième dispositif informatique de gestion 43 ne sont pas enregistrées.

Dans le cadre d'un perfectionnement de l'invention, la puce électronique primaire 33, la première et deuxiéme puces électroniques 57 et 58 sont d'une part identifiées en fonction des poches 3,7,8 concernées puis inhibées, la poche de sang mère 2 contenant l'ensemble des codes de désinhibation. Ainsi les puces électroniques 33,57,58, respectivement de la poche de sang primaire 3 (devenant successivement la poche de sang primaire 30 lorsqu'elle est filtrée et la poche de sang primaire centrifugée 40) la poche à plasma 7 et la poche d'anticoagulant 8 sont déshinibées au fur et à mesure des opérations par la transmission des codes de proche en proche ; cette façon d'opérer évite de transférer, par exemple, par inadvertance des informations destinées à la poche de sang primaire 3, 30, 40, par erreur à l'une des poches à plasma ou d'anticoagulant 7 ou 8.

## Revendications

1. Procédé de fiabilisation de la traçabilité des produits sanguins issus d'un prélèvement de sang total dans une poche de sang mère (2) et composés du plasma, d'une phase riche en globules rouges, éventuellement d'une phase contenant des plaquettes, obtenus par des procédés de filtration (1) et/ou centrifugation (4) et séparation (6) des phases, équipés de dispositifs de pilotage électronique (11,41,50), à l'aide de kits de prélèvement, dont la composition varie en fonction des produits sanguins recherchés et de la réglementation, comprenant principalement des poches (2,3,7,8,20,30,40) reliées entre elles par des tubulures (34,56,63,66) intégrant au moins un dispositif de filtration (5,67), chacune des poches (2,20,7,8,30), comportant une puce électronique (16,29,57,58,33) susceptible de mémoriser et d'échanger des informations avec un dispositif de communication électronique (15,28,32,37,42,48,52,53), constituant un moyen de recueil et de transfert d'informations, par le biais d'antennes annulaires, la centrifugation des poches se faisant par le biais d'une centrifugeuse comprenant notamment un godet de centrifugation (46) et un conteneur de centrifugation (39), la séparation du plasma et de la phase riche en globules rouges se faisant par le biais de trois berceaux (51,54 et 55) comportant chacun une porte et une paroi mobile (59,64,65) recevant respectivement les poches (40,7 et 8) équipées respectivement de la puce électronique primaire (33) de la première puce électronique (57) et de la deuxième puce électronique (58), le processus de chaque procédé (1,4,6) intégrant des moyens informatiques (13,36,43) de recueil et de traitement d'informations provenant de la poche de sang à traiter (2,30,40,8) et provenant du procédé lui-même et de ses opérateurs, ces moyens informatiques (13,36,43) étant associés à des moyens de fiabilisation (9,19,22,44 et 45,68,70,51,48,54,52,55,53) du recueil et du transfert d'informations vers la ou les poches (30,40,8) contenant le produit sanguin transformé.

2. Procédé selon la revendication 1, **caractérisé en ce que** la filtration (1) se fait en suspendant la poche de sang mère (2,20), reliée à une poche de sang primaire (3,30) par une deuxième tubulure, un premier dispositif de filtration (5) et une troisième tubulure (34), dont le contenu de la puce mère (16) a été lu préalablement par le premier dispositif de communication électronique (15) et enregistré par le dispositif informatique de gestion (13) qui recueille en cours de filtration des informations provenant du dispositif de pilotage électronique (11) et des opérateurs et les retransmet, lorsque la poche de sang mère (20) est vide, vers la puce électronique primaire (33) de la poche de sang primaire (30) de sang filtré avant sa séparation, par un dispositif de soudure-coupe (22), de la poche de sang mère (20) vide et du premier dispositif de filtration (45).

3. Procédé selon les revendications 1 et 2, **caractérisé en ce qu'**un moyen de fiabilisation consiste à affecter un crochet de suspension (9,19), fixe ou mobile, muni d'un moyen de fermeture (10,24), à chaque poche de sang mère (2) à filtrer, le moyen de fermeture (10) étant ouvert lorsque le crochet de suspension (9) est au poste d'accrochage (69) en attente d'accrochage de la poche de sang mère (2) à filtrer, puis est refermé pendant toute la phase de filtration pour s'ouvrir à nouveau lorsque le crochet de suspension (19) est au poste de déchargement (21) et permettre le décrochage de la poche de sang mère (20) vide, le crochet de suspension (19) étant ensuite refermé en attente d'une nouvelle affectation lorsque les informations ont été transmises à la puce électronique primaire (33).

4. Procédé selon la revendication 3, **caractérisé en ce que** l'ouverture du crochet de suspension (9) arrivant à un poste d'accrochage (69) se fait par basculement du dispositif d'ouverture et de fermeture (10), à l'aide d'un premier dispositif électromécanique (12) qui tend un ressort hélicoïdal (17), l'accrochage de la poche de sang mère (2) entraînant la refermeture du crochet de suspension (9) par abaissement de ce dernier par compression du ressort à boudin (18) et détente du ressort hélicoïdal (17) qui rabat le dispositif d'ouverture et de fermeture (10), l'ouverture du crochet de suspension (19) au poste de déchargement (21) se faisant sous l'action d'un deuxième dispositif électromécanique (23) appuyant sur le dispositif d'ouverture et de fermeture (24) en mettant le ressort hélicoïdal (26) sous tension, sa refermeture étant effectuée par détente du ressort hélicoïdal (26) au niveau du poste de déchargement par retrait du deuxième dispositif électromagnétique (23) et/ou échappement du crochet de suspension (19) lorsque ce dernier quitte le poste de déchargement (21) et remonte à sa position initiale sous l'action de détente du ressort à boudin (25) en attente d'une nouvelle affectation.

5. Procédé selon les revendications 2 et 3, **caractérisé en ce que** l'appareillage de soudure-coupe (22) comprend un premier dispositif de soudure-coupe (35) qui assure la fermeture par soudure et par une coupe située au milieu de la soudure et comporte un premier alvéole (27), dans lequel on place la poche de sang mère (20) vide, qui est muni d'un deuxième dispositif de communication électronique (28) qui lit à nouveau le contenu de la puce électronique mère (29) de la poche de sang mère (20) vide et identifie, dans le premier dispositif informatique de gestion (13), les informations venant de la puce électronique mère (16) et les informations complémentaires affectées à la poche de sang mère (2,20) par l'intermédiaire des crochets de suspension (8.19) affectés et les transmet dans la puce électronique primaire (33) de la poche de sang primaire (30) qui est placée dans un deuxième alvéole (31) muni d'un troisième dispositif de communication électronique (32), l'opération de soudure-coupe, par le premier dispositif de soudure-coupe (35), n'étant enclenchée, par le dispositif électronique de pilotage (11), que lorsque l'ensemble des informations a été correctement transmis par le dispositif informatique de gestion (13).

6. Procédé selon la revendication 1, **caractérisé en ce que** la centrifugation d'une poche de sang primaire (30) est précédée, d'une part par le transfert, grâce à un quatrième dispositif de communication électronique (37), des informations contenues dans la puce électronique primaire (33) vers un deuxième dispositif informatique de gestion (36) qui est intégré au dispositif de centrifugation (4), d'autre part par son introduction dans un conteneur de centrifugation (39) équipé de moyens d'identification (45,68) permettant d'affecter les informations complémentaires concernant les opérateurs et les opérations de centrifugation à celles provenant de la puce électronique primaire (33), afin de pouvoir les retransférer, après centrifugation dans la puce électronique (33) de la poche de sang primaire centrifugée (40), grâce à un sixième dispositif de communication (42).

7. Procédé selon la revendication 6, **caractérisé en ce qu'**un moyen d'identification du conteneur de centrifugation (39) est constitué par une puce électronique de conteneur (45), qui contient des informations concernant des critères d'identification qui sont spécifiques au conteneur de centrifugation (39) considéré et dans laquelle un cinquième dispositif de communication électronique (44) introduit les caractéristiques d'identification de la poche de sang primaire (30) qu'il contient, un autre moyen d'identification étant constitué par un détrompeur (68) obligeant le conteneur de centrifugation (39) à être toujours placé dans le même godet de centrifugation (46), ces deux moyens d'identification permettant d'affecter à la poche de sang primaire centrifugée (40) les informations concernant le godet de centrifugation (46) et le conteneur de centrifugation (39).

8. Procédé selon la revendication 1, **caractérisé en ce que** la séparation des phases est réalisée par un dispositif de séparation (6) qui comporte un troisième dispositif informatique de gestion (43) relié à un dispositif de pilotage électronique (50) et relié respectivement à un septième, huitième et neuvième dispositifs de communication électronique (48,52,53), disposés dans la porte de chargement respectivement des premier, deuxième et troisième berceaux (51,54,55), recevant respectivement la poche de sang primaire centrifugée (40) la poche à plasma (7) et la poche d'anticoagulant (8), la totalité des informations contenues dans la puce électronique primaire (33) de la poche de sang primaire centrifugée (40) étant recopiée dans le troisième dispositif informatique de gestion (43) à l'aide du septième dispositif de communication électronique (48), les informations résultant du cycle de séparation des phases et provenant de la poche d'anticoagulant (7) étant intégrées dans le troisième dispositif informatique de gestion (43) puis l'ensemble des informations réunies étant, d'une part transmis vers la puce électronique primaire (33) et d'autre part transmis vers la poche à plasma (7).

9. Procédé selon l'une quelconque des revendications 1,2,5,6,7, **caractérisé en ce qu'**un même dispositif de communication électronique peut servir à la place de plusieurs dispositifs de communication électroniques (15,28,32,37,42,48,52,53).

10. Procédé selon la revendication 1, **caractérisé en ce que** les puces électroniques (57,58) sont identifiées comme étant associées à une poche de sang primaire (3,30,40) à une poche à plasma (7) et à une poche d'anticoagulant (8) puis inhibées, la poche de sang mère (2,20) contenant l'ensemble des codes de désinhibation de manière que les puces électroniques (33,57,58) de la poche de sang primaire (3,30,40) de la poche à plasma (7) et de la poche d'anticoagulant (8) soient déshinibées au fur et à mesure des opérations par la transmission, de proche en proche, des codes par le biais des dispositifs de communication électroniques (15,32,48,52,53).

## Claims

1. A method for enhancing the reliability of the traceability of blood products resulting from a sample of whole blood in a mother blood bag (2) and plasma compounds from a red blood cells rich phase, or possibly a platelet-containing phase, obtained by methods for filtering (1) and/or centrifuging (4) and separating (6) the phases, provided with electronic control devices (11, 41, 50), using sampling kits, the composition of which varies as a function of the desired blood products and the rules, mainly including bags (2, 3, 7, 8, 20, 30, 40) connected together by pipes (34, 56, 63, 66) integrating at least one filtering device (5, 67), with each one of the bags (2, 20, 7, 8, 30) including an electronic chip (16, 29, 57, 58, 33) liable to store and exchange information with an electronic communication device (15, 28, 32, 37, 42, 48, 52, 53) composing means for collecting and transferring information, through annular antennas, with the centrifuging of the bags being carried out with a centrifuging machine, more particularly including a centrifuging cup (46) and a centrifuging container (39), with the separation of plasma and the red blood cells rich phase being carried out in three cradles (51, 54 and 55), each one including a door and a mobile wall (59, 64, 65) and respectively receiving the bags (40, 7 and 8) respectively provided with the primary electronic chip (33), the first electronic chip (57) and the second electronic chip (58), with the process of each method (1, 4, 6) integrating computer means (13, 36, 43) for collecting and processing information issued by the blood bag to be processed (2, 30, 40, 8) and coming from the method itself and the operators thereof, with such computer means (13, 36, 43) being associated with means for enhancing the reliability (9, 19, 22, 44 and 45, 68 70, 51, 48, 54, 52, 55, 53) during the collecting and transferring of information to the bag(s) (30, 40, 8) containing the transformed blood product.

2. A method according to claim 1, **characterized in that** the filtration (1) is performed by suspending the mother blood bag (2, 20), connected to a primary blood bag (3, 30) by a second tube, with a first filtering device (5) and a third tube (34), the content of the mother chip (16) of which has been read beforehand by the first electronic communication device (15) and recorded by the management computer device (13) which collects, during the filtration, information issued by the electronic control device (11) and the operators and transmits it back, when the mother blood bag (20) is empty, to the primary electronic chip (33) of the primary blood bag (30) of blood filtered prior to being separated, by a device welding-cutting (22) the empty mother blood bag (20) and the first filtering device (45).

3. A method according to claims 1 and 2, **characterized in that** the means for enhancing the reliability consists in assigning a stationary or mobile suspension hook (9, 19) provided with closing means (10, 24) to each mother blood bag (2) to be filtered, with the closing means (10) remaining open when the suspension hook (9) is at the suspension station (69), awaiting the mother blood bag (2) to be filtered, to be suspended and is then closed again during the whole filtration phase, to be opened again when the suspension hook (19) is at the unloading station (21) and thus enable the unhooking of the empty mother blood bag (20), with the suspension hook (19) being then closed again, awaiting a new assignment when information has been transmitted to the primary electronic chip (33).

4. A method according to claim 3, **characterized in that** the opening of the suspension hook (9) arriving at a hooking station (69) is obtained by switching the opening and closing device (10), using a first electromechanical device (12), which stretches a helical spring (17), with the suspension of the mother blood bag (2) causing the closing again of the suspension hook (9) by lowering the latter, via the compression of the coil spring (18) and release of the helical spring (17) which folds the opening and closing device (10), with the opening of the suspension hook at the unloading station (21) being carried out under the action of a second electromechanical device (23) pressing the opening and closing device (24) by stretching the helical spring (26), with the closing again being carried out by releasing the helical spring (26) at the unloading station, by retraction of the second electromechanical device (23) and/or the clearing of the suspension hook (19) when the latter leaves the unloading station (21) and travels up back to its initial position under the action of the release of the coil spring (25) awaiting a new assignment.

5. A method according to claims 2 and 3, **characterized in that** the welding-cutting device (22) includes a first welding-cutting device (35) which provides the closing by welding and by cutting in the middle of the welding, and includes a first cell (27) wherein the empty mother blood bag (2) is placed, which is provided with a second electronic communication device (28), which reads again the contents of the mother electronic chip (29) of the empty mother blood bag (2) and identifies, in the first computer management device (13) information coming from the mother electronic chip (16) and additional information assigned to the mother blood bag (2, 20) through assigned suspension hooks (8, 19) and transmit it into the primary electronic chip (33) of the primary blood bag (30) which is placed in a second cell (31) provided with a third electronic communication device (32), with the welding-cutting operation executed by the first welding-cutting device (35) being triggered by the electronic control device (11) only when all information has been correctly transmitted by the management computer device (13).

6. A method according to claim 1, **characterized in that** the centrifuging of a primary blood bag (30) is preceded, on the one hand, by the transfer, through a fourth electronic communication device (37), of the information contained in the primary electronic chip (33) to a second management computer device (36) which is integrated to the centrifuging device (4), and on the other hand through the introduction thereof into a centrifuging container (39) provided with identification means (45, 68), thus making it possible to assign the additional information relative to the operators and the centrifuging operation to those coming from the primary electronic chip (33), in order to be able to transmit it again, after centrifuging in the electronic chip (33) of the centrifuges primary blood bag (40), thanks to a sixth communication device (42).

7. A method according to any claim 6, **characterized in that** a means for identifying a centrifuging container (39) is composed of a container electronic chip (45), which contains information concerning identification criteria which are specific to the considered centrifuging container (39) and wherein a fifth electronic communication device (44) introduces the identification characteristics of the primary blood bag (30) it contains, another identifying means being composed of a polarizing slot (68) forcing the centrifuging container (39) to be always positioned in the same centrifuging cup (46), with both such identifying means making it possible to assign to the centrifuged primary blood bag (40) the information concerning the centrifuging cup (46) and the centrifuging container (39).

8. A method according to claim 1, **characterized in that** the separation of phases is performed by a separation device (6) which includes a third management computer device (43) connected to an electronic control device (50) and respectively connected to a seventh, eighth and ninth electronic communication device (48, 52, 53), positioned in the loading door, respectively of the first, second and third cradles (51, 54, 55), respectively receiving the centrifuged primary blood bag (40), the plasma bag and the anticoagulant bag (8), with the whole information contained in the primary electronic chip (33) of the centrifuged primary blood bag (40) being copied into the third management computer device (43) using the seventh electronic communication device (48), with the information resulting from the phases separation cycle and coming from the anticoagulant bag (7) being integrated in the third management computer device (43) then all the information collected being, on the one hand, transmitted to the primary electronic chip (33) and on the other hand transmitted to the plasma bag (7).

9. A method according to any one of claims 1, 2, 5, 6, 7, **characterized in that** the same electronic communication device can be used instead of several electronic communication devices (15, 28, 32, 37, 42, 48, 52, 53).

10. A method according to claim 1, **characterized in that** the electronic chips (57, 58) are identified as being associated with a primary blood bag (3, 30, 40), with a plasma bag (7) and an anticoagulant bag (8) and then disabled, with the mother blood bag (2, 20) containing all the activating codes so that the electronic chips (33, 57, 58) of the primary blood bag (3, 30, 40) of the plasma bag (7) and the anticoagulant bag (8) can be activated when and as the operations are performed through the step-by-step transmission of the codes through the electronic communication devices (15, 32, 48, 52, 53).

## Patentansprüche

1. Verfahren zur Herstellung der Zuverlässigkeit der Rückverfolgbarkeit von aus einer Entnahme des kompletten Blutes stammenden Blutprodukten in einem Mutterblutbeutel (2) und die sich aus Plasma, einer an roten Blutkörperchen reichen Phase, eventuell einer Plättchen enthaltenden Phase, die durch Filter- (1) und / oder Zentrifugal- (4) und Trennverfahren (6) der Phasen erhalten wurden, die mit elektronischen Steuervorrichtungen (11, 41, 50) ausgerüstet sind, zusammensetzen, mithilfe von Entnahmesets, deren Zusammensetzung in Abhängigkeit von den gewünschten Blutprodukten und der Reglementierung variiert, umfassend im Wesentlichen Beutel (2, 3, 7, 8, 20, 30, 40), die untereinander durch Leitungen (34, 56, 63, 66) verbunden sind, die wenigstens eine Filtervorrichtung (5, 67) beinhalten, wobei jeder der Beutel (2, 20, 7, 8, 30) einen elektronischen Chip (16, 29, 57, 58, 33) umfasst, der geeignet ist, Informationen zu speichern und mit einer elektronischen Kommunikationsvorrichtung (15, 28, 32, 37, 42, 48, 52, 53) austauschen, die ein Mittel zur Aufnahme und Übertragung von Informationen über Ringantennen bildet, wobei das Zentrifugieren der Beutel über eine Zentrifuge erfolgt, die insbesondere einen Zentrifugenbecher (46) und einen Zentrifugenbehälter (39) umfasst, wobei die Trennung des Plasmas und der an roten Blutkörperchen reichen Phase über drei Wiegen (51, 54 und 55) erfolgt, die jede eine Tür und eine mobile Wand (59, 64, 65) umfassen, die jeweils die Beutel (40, 7 und 8) aufnehmen, die jeweils mit dem primären Chip (33) des ersten Chips (57) und des zweiten Chips (58) ausgerüstet sind, wobei der Prozess jedes Verfahrens (1, 4, 6) IT-Mittel (13, 36, 43) zur Aufnahme und zur Verarbeitung von aus dem zu verarbeitenden Blutbeutel (2, 30, 40, 8) stammenden und aus dem Verfahren selbst und seinen Operateuren stammenden Informationen, wobei diese Informationsmittel (13, 36, 43) Mittel zur Herstellung der Zuverlässigkeit (9, 19, 22, 44 und 45, 68, 70, 51, 48, 54, 52, 55, 53) der Aufnahme und der Übertragung auf den oder die das verarbeitete Blutprodukt enthaltenden Beutel (30, 48, 8) zugeordnet sind.

2. Verfahren gemäß Anspruch 1, **dadurch gekennzeichnet, dass** die Filtrierung (1) durch Aufhängen des Mutterblutbeutels (2, 20) erfolgt, der mit einem primären Blutbeutel (3, 30) durch eine zweite Leitung, eine erste Filtriervorrichtung (5)und eine dritte Leitung (34), deren Inhalt des Mutterchips (16) zuvor von der ersten elektronischen Kommunikationsvorrichtung (15) gelesen wurde und von der IT-Verwaltungsvorrichtung (13) gespeichert wurde, die im Verlauf der Filtrierung Informationen aufnimmt, die aus der elektronischen Steuervorrichtung (11) und den Operatoren stammen, und sie, wenn die Mutterbluttasche (20) leer ist, auf den primären Chip (33) des vor seiner Trennung vom leeren Mutterblutbeutel (20) und der ersten Filtriervorrichtung (45) durch eine Schneid-Schweiß-Vorrichtung (22) gefilterten primären Blutbeutels (30) weiterleitet, verbunden ist.

3. Verfahren gemäß Anspruch 1 und 2, **dadurch gekennzeichnet, dass** ein Mittel zur Herstellung der Zuverlässigkeit in der Zuordnung eines festen oder mobilen, mit einem Verschlussmittel (10, 24) versehenen Aufhängungshakens (9, 19) zu jedem zu filternden Mutterblutbeutel (2) besteht, wobei das Verschlussmittel (10) geöffnet wird, wenn der Aufhängungshaken (9) am Aufhängungsposten (69) auf das Aufhängen des zu filternden Mutterblutbeutels (2) wartet, dann während der gesamten Filtrierphase wieder verschlossen wird, um sich erneut zu öffnen, wenn der Aufhängungshaken (19) sich am Entladeposten (21) befindet und das Abhängen des leeren Mutterblutbeutels (20) zu erlauben, wobei der Aufhängungsbeutel (19) anschließend in der Erwartung einer neuen Zuordnung wieder verschlossen wird, wenn die Informationen auf den primären Chip (33) übertragen wurden.

4. Verfahren gemäß Anspruch 3, **dadurch gekennzeichnet, dass** die Öffnung des an einem Aufhängungsposten (69) eintreffenden Aufhängungshakens (9) per Kippen der Öffnungs- und Verschlussvorrichtung (10) mithilfe einer ersten elektromechanischen Vorrichtung (12) erfolgt, die eine Spiralfelder (17) hält, wobei das Aufhängen des Mutterblutbeutels (2) den erneuten Verschluss des Aufhängungshakens (9) durch Absenken desselben per Kompression der Springfeder (18) und Entspannen der Spiralfeder (17) nach sich zieht, die die Öffnungs- und Verschlussvorrichtung (10) umschlägt, wobei das Öffnen des Aufhängungshakens (19) am Entladeposten (21) unter der Wirkung einer zweiten elektromagnetischen Vorrichtung (23) erfolgt, die auf die Öffnungs- und Verschlussvorrichtung (24) drückt, indem die Spiralfelder (26) unter Spannung gesetzt wird, wobei ihr erneuter Verschluss per Entspannung der Spiralfeder (26) beim Entladeposten per Zurückziehen der zweiten elektromagnetischen Vorrichtung (23) und /oder Austritt des Aufhängungshakens (19) erfolgt, wenn dieser den Entladeposten (21) verlässt wieder unter der Entspannungswirkung der Springfeder (25) in der Erwartung einer neuen Zuordnung in seine ursprüngliche Position hochfährt.

5. Verfahren gemäß Anspruch 2 und 3, **dadurch gekennzeichnet, dass** das Schneid-Schweiß-Gerät (22) eine erste Schneid-Schweiß-Vorrichtung (35) umfasst, die den Verschluss per Schweißen und durch einen Schnitt gewährleistet, der sich in der Mitte der Schweißnaht befindet und eine erste Wabe (27) umfasst, in der der leere Mutterblutbeutel (20) platziert wird, der mit einer zweiten elektronischen Kommunikationsvorrichtung (28) versehen ist, der erneut den Inhalt des Mutterchips (29) des leeren Mutterblutbeutels (20) ausliest und in der ersten IT-Verwaltungsvorrichtung (13) die Informationen identifiziert, die vom Mutter-Chip (16) kommen, und die komplementären Informationen, die dem Mutterblutbeutel (2, 20) über zugeordnete Aufhängungshaken (8, 19) zugeordnet sind, und sie auf den primären Chip (33) des primären Blutbeutels (30) überträgt, der in einer zweiten Wabe (31) platziert ist, die mit einer dritten elektronischen Kommunikationsvorrichtung (32) versehen ist, wobei die Schneid-Schweiß-Operation durch die erste Schneid-Schweiß-Vorrichtung (35) durch die elektronische Steuervorrichtung (11) nur dann ausgelöst wird, wenn alle Informationen von der IT-Verwaltungsvorrichtung (13) ordnungsgemäß übertragen worden sind.

6. Verfahren gemäß Anspruch 1, **dadurch gekennzeichnet, dass** dem Zentrifugieren eines primären Blutbeutels (30) einerseits die Übertragung der im primären Chip (33) enthaltenen Informationen auf eine zweite IT-Verwaltungsvorrichtung (36), die in die Zentrifugalvorrichtung (4) integriert ist, dank einer vierten elektronischen Kommunikationsvorrichtung (37) und andererseits durch seine Einführung in einen Zentrifugenbehälter (39), der mit Identifikationsmitteln (45, 68) ausgerüstet ist, die die Zuordnung der komplementären Informationen bezüglich der Operatoren und die Zentrifugaloperationen zu denen erlauben, die aus dem primären Chip (33) stammen, um sie nach dem Zentrifugieren auf den Chip (33) des zentrifugierten primären Blutbeutels (40) dank einer sechsten Kommunikationsvorrichtung (42) zurückübertragen zu können, vorausgeht.

7. Verfahren gemäß Anspruch 6, **dadurch gekennzeichnet, dass** ein Identifikationsmittel des Zentrifugenbehälters (39) durch einen Chip des Behälters (45) gebildet wird, der Informationen bezüglich der Identifikationskriterien enthält, die spezifisch für den betrachteten Zentrifugalbehälter (39) sind und in dem eine fünf elektronische Kommunikationsvorrichtung (44) die Identifikationsmerkmale des primären Blutbeutels (30) einführt, die er enthält, wobei ein andere Identifikationsmittel durch einen identifizierten Steckplatz (68) gebildet wird, der den Zentrifugenbehälter (39) zwingt, stets in demselben Zentrifugenbecher (46) platziert zu sein, wobei diese zwei Identifikationsmittel die Zuordnung der Informationen bezüglich des Zentrifugenbechers (40) und des Zentrifugenbehälters (39) zum zentrifugierten primären Blutbeutel (40) erlauben.

8. Verfahren gemäß Anspruch 1, **dadurch gekennzeichnet, dass** die Trennung der Phasen durch eine Trennvorrichtung (6) realisiert wird, die eine dritte IT-Verwaltungsvorrichtung (43) umfasst, die mit einer elektronischen Steuervorrichtung (50) verbunden ist und jeweils mit einer siebten, achten und neunten elektronischen Kommunikationsvorrichtung (48, 52, 53) verbunden ist, die in der Ladetür jeweils der ersten, zweiten und dritten Wiege (51, 54, 55) angeordnet sind, die jeweils den zentrifugierten primären Blutbeutel (40), den Plasmabeutel (7) und den Antikoagulationsbeutel (8) aufnehmen, wobei alle in dem primären Chip (33) des zentrifugierten primären Blutbeutels (40) enthaltenen Informationen anhand der siebten elektronischen Kommunikationsvorrichtung (48) in die dritte IT-Verwaltungsvorrichtung (43) kopiert werden, wobei die aus dem Trennungszyklus der Phasen resultierenden und aus dem Antikoagulationsbeutel (7) stammenden Informationen in die dritte IT-Verwaltungsvorrichtung (43) integriert werden und dann, nachdem alle Informationen zusammengefasst sind, einerseits auf den primären Chip (33) übertragen werden und andererseits auf den Plasmabeutel (7) übertragen werden.

9. Verfahren gemäß Anspruch 1, 2, 5, 6, 7, **dadurch gekennzeichnet, dass** eine und dieselbe elektronische Kommunikationsvorrichtung anstelle von mehreren elektronischen Kommunikationsvorrichtungen (15, 28, 32, 37, 42, 48, 52, 53) dienen kann.

10. Verfahren gemäß Anspruch 1, **dadurch gekennzeichnet, dass** die Chips (57, 58) als einem primären Blutbeutel (3, 30, 40), einem Plasmabeutel (7) und einem Antikoagulationsbeutel (8) zugeordnet und dann als gesperrt identifiziert sind, wobei der Mutterblutbeutel (2, 20) alle Entsperrungscodes derart enthält, dass die Chips (33, 57, 58) des primären Blutbeutels (3, 30, 40) des Plasmabeutels (7) und des Antikoagulationsbeutels (8) im Verlauf der Operationen durch die Übertragung der Codes von Beutel zu Beutel¹ über die elektronischen Kommunikationsvorrichtungen (15, 32, 48, 52, 53) entsperrt werden.
